# EUROPEAN PATENT APPLICATION

(11) **EP 4 656 159 A1**
(43) Date of publication of application: **03.12.2025**
(21) Application number: 24179400.7
(22) Date of filing: 31.05.2024
(51) Int. Cl.: A61F 2/00, A61B 17/34

(54) **DELIVERY DEVICE, A SYSTEM INCLUDING THE DELIVERY DEVICE AND A METHOD OF LOADING A FLEXIBLE SHEET-SHAPED IMPLANT INTO A DELIVERY DEVICE**

(71) Applicant: ZuriMED Technologies AG, 8008 Zürich (CH)
(72) Inventor: BANZET, Pol, 8008 Zürich (CH); SOLE ESCOBAR, Carlota, 8008 Zürich (CH); SENN, Ronja, 8008 Zürich (CH); BACHMANN, Elias, 8008 Zürich (CH); LI, Xiang, 8008 Zürich (CH)
(74) Representative: Vossius & Partner Patentanwälte Rechtsanwälte mbB

(57) **Abstract**

The present invention relates to a delivery device for loading, delivering and/or implanting a flexible sheet-shaped implant. The delivery device comprises an implant delivery tool and an insertion tool. The implant delivery tool includes an implant delivery tool handle and a shaft. The shaft extends distally from the handle. The shaft is configured to receive an end portion of the sheet-shaped implant. The insertion tool includes a longitudinally extending tube. The longitudinally extending tube has an inner lumen. The inner lumen is configured to receive the shaft of the implant delivery tool rotatably. The tube of the insertion tool includes a tube slit that extends from an end of the tube for rolling the sheet-shaped implant.

## Description

The present invention relates to a delivery device, a system including the delivery device and a method of loading a flexible sheet-shaped implant into a delivery device.

Tendons, and other musculoskeletal soft tissues may rupture, tear partially or completely which can result in need of surgery. The repair procedure can be done with large incisions or with arthroscopic techniques. To repair such structures, the surgeon may use anchors and sutures to reattach the tendon to the bone. The repaired area may then be covered with a graft to facilitate healing. However, during arthroscopic surgery, it can be challenging to insert an implant (e.g., the graft) through a small incision in the skin or through a cannula and correctly position it in the desired location. It is also challenging to protect the implant from damage when inserting it as described above. In addition, it may be challenging to reliably and compactly load the implant.

A device for delivering implants is disclosed in US 11,259,939 B2. US 11,259,939 B2 discloses an implant delivery device that includes an implant holding portion configured to retain a sheet-shaped implant. The implant is passed through a cannula with a tapered intermediate portion to roll up the implant. However, during the rolling described in US 11,259,939 B2 the implant may fold or even crinkle and even get stuck in the cannula. A reliable compression may depend on the skill of a user. Further known implant delivery devices are disclosed in US 2019/0336261 A1, US 2023/0021001 A1, and US 2023/0149151 A1.

The present invention intends to address one or more of the above challenges. In particular, the present invention provides devices and methods that facilitate loading and/or delivering and/or unloading an implant. Further advantages of the present invention may be that an easy loading mechanism is provided, that the device may be reusable, and that the device may be manually actuated. Further, the device may be compatible with a range of implants.

A first aspect of the present invention relates to a delivery device for loading and/or delivering and/or implanting a flexible sheet-shaped implant. The delivery device may comprise an implant delivery tool and/or an insertion tool. The implant delivery tool includes an implant delivery tool handle and a shaft. The shaft extends distally from the handle. The shaft is configured to receive an end portion of the sheet-shaped implant.

The insertion tool includes a longitudinally extending tube. The longitudinally extending tube has an inner lumen. The inner lumen is configured to receive the shaft of the implant delivery tool rotatably. The tube of the insertion tool includes a tube slit that extends from an end of the tube for rolling the sheet-shaped implant.

The present application provides a novel device for delivering an implant into the body to its target site and holding it in position while it is being fixated.

In particular, as will be described in greater detail below, the shaft receives, i.e., holds, the implant and then, upon being positioned in the tube slit of the tube, the shaft can be rotated, and the implant may be rolled around the shaft. This provides for a simple yet effective compression of the implant allowing delivery through a cannula or directly through an incision (i.e. an arthroscopic portal). The sheet-shaped implant may be rolled in an even manner (e.g., without wrinkles or folds).

The term sheet-shaped as used herein may mean that the implant has a substantially planar shape having a thickness. For example, a thickness may be less than 1/10 of a width and length. The sheet-shaped implant may have any outline, e.g. rectangular, polygonal, round, angled (e.g. "L"-shape), etc.

The system comprises two devices that work together: the delivery tool and the insertion tool. The system can be made to be reusable or disposable. The delivery tool is comprised of a handle and shaft which has an opening to accept the implant at its distal end. The insertion tool is comprised of a handle and tube which serve to roll the implant and protect it as it is delivered to the target site.

The delivery device may be a medical device. The flexible sheet-shaped implant may be made of or comprise a woven or nonwoven material or a graft. In one example, the implant may be or at least comprise a sheet of, e.g. a patch of, woven or nonwoven material. For example, the implant may be a rectangular felt having a thickness of e.g. 1 mm. The implant delivery tool and the insertion tool may be separable from each other. One or both of the tools mentioned herein may be a handheld device that is manually operated. In some embodiments, the delivery device as described herein may also comprise motors and controllers for some or all of the method steps described herein. The device may also be provided as a single compact device having the tools as components thereof, e.g., in a housing.

The tube slit may extend from a distal or proximal end of the tube. The tube may partially or fully encompass the shaft. The tube and/or the shaft may be round, in particular circular.

Preferably, the shaft is defined by a shaft wall. The shaft may include a first longitudinally extending shaft slit in the shaft wall. For example, the shaft may have a tubular shape. The shaft slit may hold the flexible implant, in particular for positioning the implant in the tube slit and for rolling the implant around the shaft. The shaft wall may define the tubular shape.

Preferably, the first shaft slit extends from a distal end of the shaft. Additionally or alternatively, the first shaft slit may extend from a proximal end of the shaft. If the shaft slit is arranged at the distal end, the implant may be held at a distal end improving handling during insertion through a cannula or directly through an incision (i.e. an arthroscopic portal). Generally, the implant delivery tool may be configured to hold the implant at a distal end portion.

Preferably, the first shaft slit extends exclusively in the longitudinal direction of the shaft. Thereby, the implants can be easily inserted onto the shaft.

The first shaft slit may extend in a longitudinal and circumferential direction of the shaft. The first shaft slit may be curved and may have a helical shaft. This may improve retention of the implant in the slit.

Preferably, the shaft wall includes a radially outwardly and/or radially inwardly extending wall defining a portion of the first shaft slit. Thereby, the implant is held in between a larger surface allowing for a better retention of the implant.

Preferably, the shaft includes a second shaft slit at a radially opposing side of the first shaft slit. In some embodiments, the shaft may include only one shaft slit. The second shaft slit may also be positioned at any position in between the radially opposing side and the first shaft slit. The second shaft slit may facilitate attaching the implant to the delivery tool. The second shaft slit may have any of the features described with reference to the first shaft slit.

Preferably, the first shaft slit has a U-shape. A U-shape may increase retention of the implant in the shaft slit. Alternatively, the first shaft slit has a C shape and/or the slit may be formed by cutting a longitudinally extending hole in the tubular shaft. The first shaft slit may include a notch or projection for holding the implant. The notch or projection may extend along an axial direction of the shaft. The notch or projection may additionally improve a retention of the implant in the shaft slit.

A distal end portion of the shaft may include a clamping mechanism for gripping the implant. The clamping mechanism may include one or more teeth for gripping the implant. The clamping mechanism may allow for a reliable retention of the implant at a distal end portion of the shaft. Teeth may further keep the implant in place.

Preferably, the tube of the insertion tool extends around the shaft of the implant delivery tool and is arranged to slide on the shaft along a longitudinal axis of the shaft. The insertion tool may be configured to slide from a first position to a second position on the shaft. In the first position, the tube slit, and the distal end configured to receive an end portion of the sheet-shaped implant may be axially aligned. In this position, the implant delivery tool may be rotated to roll up the implant in the tube slit. The first position may be a loading position. In a second position (also delivery position), the insertion tool may be in a proximal end position. In the second position, the distal end configured to receive an end portion of the sheet-shaped implant may extend out of the tube.

In a variation, the distal end configured to receive an end portion of the sheet-shaped implant may be held within the tube in the second position. Additionally, the tube slit may be aligned with the distal end configured to receive an end portion of the sheet-shaped implant. In this case, the first and second position may be identical.

The insertion tool and the delivery tool may be provided as a single device or as a set of tools that may or may not be separable from each other.

Preferably, the shaft and/or the tube comprises or is made of at least one of the following materials: stainless steel, nitinol, PEEK, PPSU, or PEI. Stainless steel is preferred.

Preferably, the insertion tool includes an insertion tool handle for gripping the insertion tool. This allows holding the insertion tool in place while rotating the delivery tool handle manually.

Preferably, the insertion tool handle may have a body with a round shape for manual gripping and removing the insertion tool. A longitudinal axis of the handle may extend along a longitudinal axis of the tube. This may allow for a convenient handling when sliding and/or rotating the insertion tool.

The insertion tool handle may also extend in a radial direction or in an axial direction of the tube. Extending along a radial direction may mean that a longitudinal axis of the insertion tool handle extends in the radial direction, i.e. radially outwardly. Such an insertion tool handle may allow for a precise navigation when inserting the tube and/or the shaft into a cannula.

Preferably, the insertion tool handle and/or the implant delivery tool handle comprises or is made of at least one of the following materials: silicone, aluminum, stainless steel, carbon fiber, PEEK, PPSU, PEI, PP-HS, POM, PC, and ABS. Silicone may be preferred.

The shaft may include one or more bends, in particular to improve in situ navigation with a loaded implant. The bends can be flexible. For example, the bends may be made of nitinol or another elastic or shape-memory material. The bends may include cut-outs in the shaft to increase flexibility of the bending section.

Preferably, a distal end of the tube and/or a distal end of the shaft includes an inclined end face. The inclined end face may move soft-tissue aside and/or penetrate soft tissue. Further, the inclined end face may help in loading implant to the shaft.

Preferably, the implant delivery tool handle includes an actuator. The actuator may be a slider. The implant delivery tool may include an implant gripper. The actuator may be connected to the implant gripper. Actuation of the actuator may be configured to cause the gripper to grip the implant and/or move the implant along the longitudinal direction of the shaft. Thereby, the implant may be securely held, and inadvertent release of the implant can be prevented.

Preferably, a relative position of the tube and the shaft can be locked. In one example, the tube and the shaft include interlocking features such as latches, levers, a snap-in, or a slide-on lock. Thereby, the implant can be securely delivered without inadvertent movement of the tube and the shaft relatively to each other.

Preferably, the implant delivery tool and/or the insertion tool comprises a bobbin. The bobbin may be arranged at a proximal part of the delivery tool and/or the insertion tool. Sutures connected to the sheet -shaped implant may be wrapped around the bobbin.

Thereby, the implant may be securely held at the distal end of the shaft and/or sutures needed during implantation can be securely stored.

Preferably, the implant delivery tool handle and/or insertion tool includes a body. The body may define a preferably longitudinal extending channel for sutures connected to the sheet - shaped implant. The longitudinally extending channel may or may not be identical to the tube slit. The channel may guide the sutures to the bobbin. Further, the channel may allow for convenient handling to avoid user distractions due to the sutures.

Preferably, the implant delivery tool and/or the insertion tool includes a clamp to grip sutures connected to the sheet -shaped implant. The clamp may be used alternatively (or additionally) to the bobbin.

Preferably, the bobbin or clamp is connected to the delivery device handle or the insertion tool handle with an elastic element, e.g. a spring. The elastic element may be configured to tension sutures connected to the sheet-shaped implant and held by the bobbin or clamp. This prevents sutures from loosely hanging around the device and distracting a user during handling.

Preferably, the tube slit of the insertion tool extends from a proximal end of the tube and/or from the distal end of the tube. Further, the tube slit may extend from the distal end of the tube to the proximal end of the tube. Alternatively, the tube slit only extends along a portion of the tube length.

A further aspect of the present invention relates to a system including the delivery device as described above and a sheet -shaped implant.

Preferably, the implant includes a first edge region. The edge region may form an end portion of the sheet-shaped implant. The edge region may include a notch or projection or trough. The notch or projection or trough may extend along the edge region of the implant. The notch or projection or trough may improve the retention of the implant in a shaft slit as described above.

Preferably, the shaft slit of the shaft has at least a length and/or width of the implant. Thereby, an entire edge or entire side of the implant may be received within the shaft slit preventing inadvertent release of the implant from the shaft slit. Further this may improve the rolling and further avoid wrinkles.

In certain embodiments, the implant may comprise a shape memory component, for example, a wire, e.g. a nitinol wire, or a spring biasing the implant to an open configuration. For example, the shape memory component (e.g. nitinol wires or sheaths) can be weaved into the implant in the center or at the edges of the implant. The shape memory component may have a circular or rectangular cross-section. The implant can be attached to the nitinol wire directly or it can be attached to the nitinol via sutures. In this manner, a shape memory material can be rolled with the patch using the insertion tool. This may help in unrolling the implant as the shape memory component may force a rolled implant out of the tube slit and/or out of a rolled configuration.

The shape memory component may have, along the plane of the sheet-shaped implant, a T-shape, follow a straight line, a diagonal line, a curved line, etc. The shape memory component may be released from the implant when the delivery device is pulled out. For example, sutures may be attached to the shape memory component. Pulling the respective sutures may pull the shape memory component out of the implant, e.g. the nitinol may be weaved out. In an example method, once the patch is securely attached to the tendon, the shape memory component (e.g. nitinol wire) is released from the patch and the delivery device is pulled out together with the shape memory component.

A further aspect of the present invention relates to a method of loading a flexible sheet-shaped implant into a delivery device. The method includes the following steps:
a. Receiving a sheet-shaped implant on a distal end of a shaft of an implant delivery tool; and
b. Moving the distal end of the shaft with the implant into a tube slit at an end of a tube of an insertion tool; and
c. Rotating the shaft of the implant delivery tool around its axis relatively to the tube and thereby drawing the implant into the tube through the tube slit and rolling the implant around the shaft.

As described above, this provides for a simple yet effective compression of the implant allowing delivery through a cannula or incision. The sheet-shaped implant may be rolled in an even manner (e.g. without wrinkles or folds).

Preferably, the receiving step includes receiving the sheet-shaped implant in a shaft slit of the shaft of the delivery tool. Thereby, the implant may be securely held at a distal end of the implant delivery tool while at the same time allowing for a simple release after the implant has been fixated in a patient.

Preferably, the receiving step includes pulling sutures that are affixed to the implant to draw the sheet-shaped implant, preferably an edge of the sheet-shaped implant, into the shaft. Thereby, the implant may be loaded without contaminating or damaging the implant.

Preferably, the receiving step includes pushing or pulling the implant into the shaft slit using a clamp or other tool.

The implant may be sutured to the shaft. For example, the implant may be folded around and sutured to another portion of the implant. Alternatively, the sutures may extend through holes of the shaft. This may allow for a particularly secure connection between the shaft and the implant.

Preferably, the step of moving the distal end of the shaft with the implant into the tube slit comprises sliding the shaft with the implant until the insertion tool reaches an end position defined by the implant delivery tool. The end position is preferably defined by a handle of the implant delivery tool.

Preferably, after rotating the shaft of the implant delivery tool and drawing the implant into the tube, an edge of the implant extends out of the tube (i.e. in the delivery position). For example, the edge may extend about one millimeter out of the tube slit. This may facilitate unrolling, i.e., delivering the implant after passing through a cannula. Alternatively, the shaft may be rotated until the implant is fully within the tube. Preferably, the step of unloading may comprise unloading the implant through the tube slit.

Preferably, the method additionally comprises the step of unloading the sheet-shaped implant. Unloading may comprise rotating the shaft of the implant delivery tool around its axis relatively to the tube. For example, the shaft may be rotated in a direction opposite to the rotation for loading the implant. A user may unload the device to exchange the loaded implant or to deliver the implant after the device has passed through the cannula.

Preferably, the shaft with the implant is pushed through the tube of the insertion tool, e.g. prior to unloading, and a distal portion of the shaft including the implant may leave the tube of the insertion tool, e.g. at a distal end of the tube.

Example embodiments of the present invention are described with reference to the following nonlimiting drawings:
Figure 1A shows a delivery device according to the present invention in an exploded view.
Figure 1B shows the delivery device of figure 1 A in an assembled form.
Figure 2A shows a tip region of an implant delivery tool of the delivery device of figure 1A and an implant in a first step of loading the implant.
Figure 2B shows the delivery device of figure 1A in a second step of loading the implant.
Figure 2C shows the delivery device of figure 1A in a first step of delivering the implant.
Figure 2D shows the delivery device of figure 1A in a second step of delivering the implant.
Figure 3 shows the delivery device of figure 1A and a felting device in a third step of delivering the implant.
Figure 4 shows the delivery device and a detailed view of a tip portion of the implant delivery tool.
Figure 5A shows a second embodiment of an insertion tool.
Figure 5B shows a second embodiment of an implant delivery tool.
Figure 5C shows the insertion tool and the implant delivery tool of the second embodiment in an assembled form forming a delivery device according to the second embodiment.
Figures 6A to 7C show a loading and a delivery of an implant with the second embodiment of the delivery device.
Figure 8A shows a side view of the insertion tool of figure 1A.
Figure 8B shows a top view of the insertion tool of figure 8A.
Figures 8C and 8D show alternative methods of loading the implant into the delivery tool.
Figures 9A to 9C show alternative methods of fixating the implant to the delivery tool.
Figure 9D shows an alternative tip portion of the implant delivery tool.
Figure 10 shows an alternative slit configuration of the implant delivery tool.
Figures 11A to 11C show further alternative slit configurations of the implant delivery tool.
Figures 12A to 12B show alternative fixation methods of the implants to the implant delivery tool.
Figure 12 C shows a slider for actuating the implant delivery tool.
Figures 13A and 13B show a fixation of sutures to the implant delivery tool of the second embodiment.

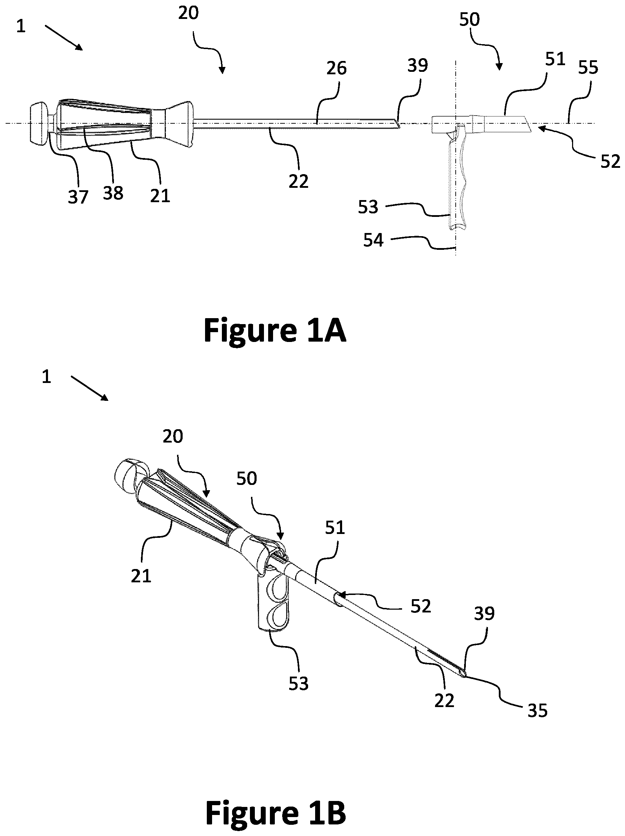

A first embodiment of the present invention is shown in figure 1A. Figure 1A shows a delivery device 1. The delivery device 1 comprises an implant delivery tool 20 and an insertion tool 50. The implant delivery tool 20 comprises an implant delivery tool handle 21 and a shaft 22. The implant delivery tool 20 has a longitudinal axis 26 and the shaft 22 and/or the delivery tool handle 21 may be substantially rotationally symmetric about the longitudinal axis 26. The shaft 22 is attached to a proximal end of the delivery tool handle 21 and may have a tubular shape. In other embodiments, the shaft 22 may have a solid cross-section.

The delivery tool handle 21 may include a delivery tool gripping portion 38. The gripping portion 38 may include longitudinally extending ridges for improving a grip of a user. Additionally our alternatively, the gripping portion may have any enhancements, such as a roughened surface, dimples and/or humps that may improve a manual grip of a user.

During use, as will be described in further detail below, a user may rotate the delivery tool 20 around the longitudinal axis 26 to load and/or unload an implant. The gripping portion 38 allows a user to rotate the delivery tool 20 around the longitudinal axis 26.

Further, the delivery tool handle 21 includes at its proximal end a bobbin 37 for securing sutures attached to an implant as will be described below.

The insertion tool 50 includes a longitudinally extending tube 51 and an insertion tool handle 53. The insertion tool handle 53 is attached to the tube 51. In the first embodiment of the present invention, the insertion tool handle 53 extends in a radial direction 54 perpendicular to a longitudinal axis 55 of the tube 51. However, as will be apparent from the second embodiment, the insertion tool handle 53 may also extend around the tube 51 or may be arranged proximally to the tube 51. The handles 21 and 53 may be made of silicone (although any of the other materials mentioned above could also be used). The shaft 22 and the tube 51 may be made of stainless steel (although any of the other materials mentioned above could also be used).

Generally, the directions proximal and distal as used herein refer to the perspective of a user during intended use of the device. Proximal may mean a direction that is closer to the user. Distal may mean a direction that is further away from the user. For example, the end 52 as indicated in figures 1A and 1B is a distal end of the insertion tool 50.

Figure 1B shows the delivery device 1 comprising the implant delivery tool 20 and the insertion tool 50 in an assembled form. In an assembled form, the implant delivery tool 20, in particular the shaft 22 of the implant delivery tool 20 is inserted into the tube 51 of the insertion tool 50. The tube 51 can slide along the longitudinal direction 26 of the shaft 22 and the shaft 22 can rotate within tube 51. The shaft 22 may have a length of 5 - 30 cm, preferably 10 - 25 cm and most preferably 14 - 20 cm. The length may be chosen based on an access port, a cannula length, and a size of the implant.

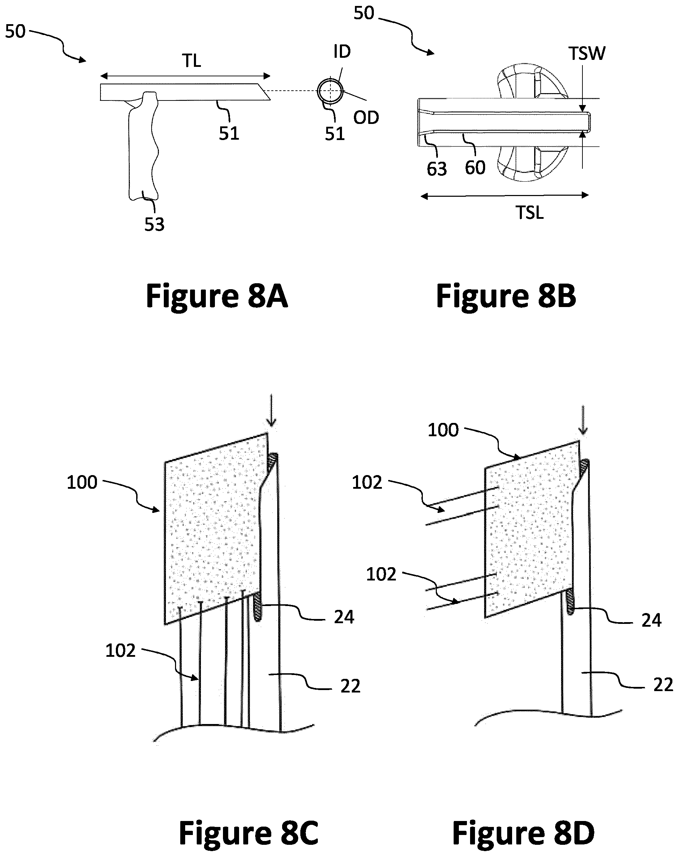

The shaft 22 may have a length that is greater than the length of the tube 51. Thereby, the shaft 22 may extend out of the distal end 52 of the tube 51 for release of the implant (as described in further detail below). For example, the tube 51 may have a tube length TL (see e.g. figure 8A) of 1 - 15 cm, preferably 4 - 10 cm and most preferably 6 - 9 cm. Preferably, the shaft 22 is at least 2, 3, 5 or 10 cm longer than the tube 51.

In some embodiments, the insertion tool 50 may be locked in a rotational position in relation to the delivery tool 20, e.g. through a form-fit. For example, the delivery tool handle 21 and the insertion tool handle 53 may include interlocking features, such as teeth or a recess (or a press-fit) in the delivery tool handle 21, that hold the insertion tool handle 53 in a fixed position when it is held in a proximal end position as e.g. shown in figure 1B.

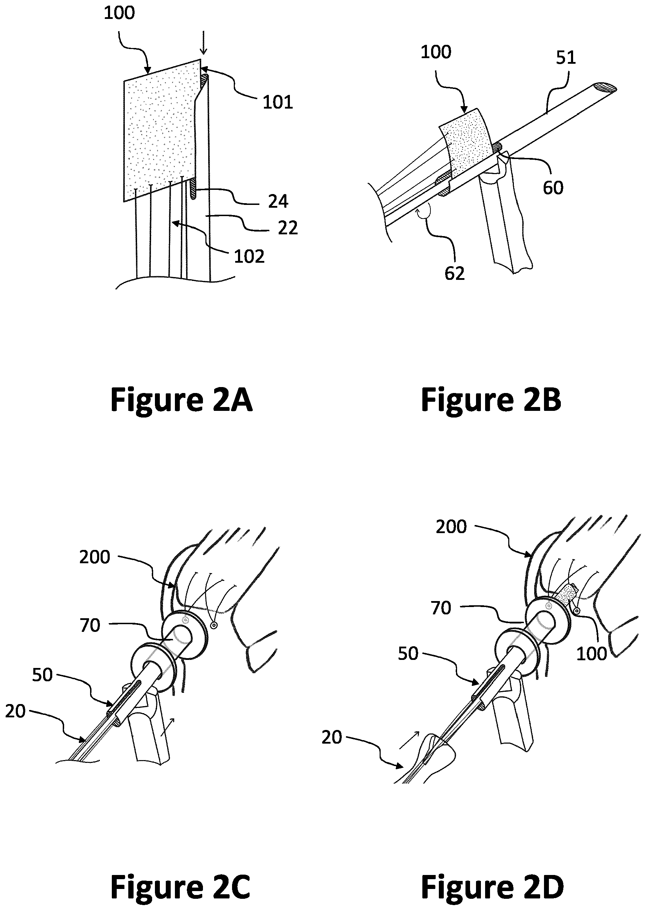

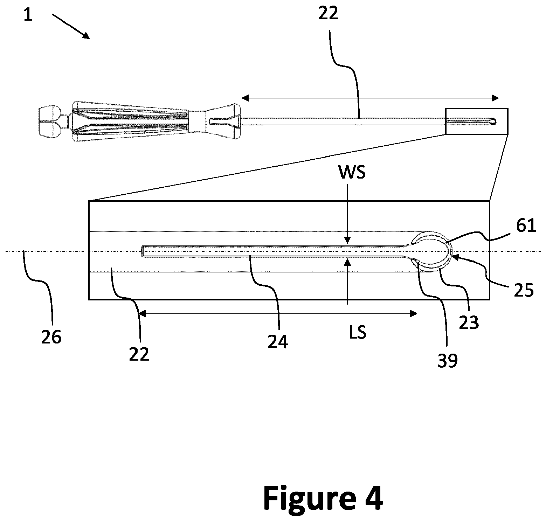

Figures 2A to 3 show how the above described implant delivery tool 20 and the insertion tool 50 may be used in loading and delivering an implant 100. The implant 100 may have a rectangular shape as shown e.g. in figure 2A. However, in other embodiments, the implant 100 may have any other shape. The implant 100 includes an edge 101 (also edge region or edge portion herein). In a first step, the implant 100 is loaded at a distal tip portion of the shaft 22. The shaft 22 includes a slit 24 that is shown in greater detail in figure 4. The edge 101 is drawn into the slit 24 such that a portion adjacent edge 101 is held within the slit 24. The implant 100 may be loaded using sutures 102 that are attached to the implant. In certain embodiments, the sutures may extend in the longitudinal direction 26 which allows pulling the implant 100 in the longitudinal direction and along the slit 24. In other embodiments as will be explained with reference to figures 8C to 9B below, the implant 100 is fixated in a different manner to the shaft 22.

According to a first example, the implant 100 may be positioned distally to a distal end of the shaft 22 and then drawn into the slit 24 in the longitudinal direction 26. In a second example, the implant 100 may be positioned radially adjacent to the slit and then pushed into the slit 24 in the radial direction of the shaft 22.

In a preferred embodiment, the slit 24 has a width that may be similar to the width of the implant 100 (at least in the region adjacent to edge 101) or slightly less. Thereby, the slit 24 holds the edge 101 of the implant 100. Thus, preferably, the implant 100 is loaded by pulling it in a proximal direction from a distal end 61 of the shaft 22 as this allows for a quicker loading.

As can be seen in figure 4, the slit 24 extends from a distal end 61 of the shaft 22. The slit 24 may have a length LS that may be 5 - 100 mm, preferably 10 - 50 mm and most preferably 25 - 35 mm. The slit 24 may have a length of at least 10, 20, or 30 mm. The slit 24 may have a length that is at least the length of an edge (e.g. edge 101) of the implant 100 that is to be slid into the slit 24. In other embodiments, the slit length may be less than the length of the edge.

Additionally, the slit 24 includes an opening 39 that tapers in the proximal direction of the slit 24 (see figure 4). This enables inserting the edge 101 of the implant into the slit 24. Also this may compress the edge of implant 100 improving a hold of the implant 100 of the slit 24.

In some embodiments a width WS of the slit 24 (see e.g. figure 4) may be 0.1 - 5 mm, preferably 0.3 - 3 mm and most preferably 0.5 - 2 mm. The slit 24 may have a width of at most 5 mm, 3 mm, 2 mm or 1.5 mm. The slit 24 may further have a width that corresponds to a width of the implant 100, e.g. have a width that is at most the thickness of an implant, preferably less than a thickness of the implant.

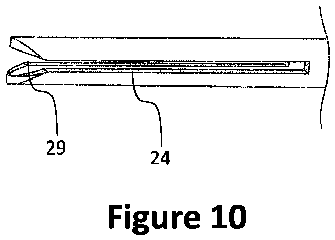

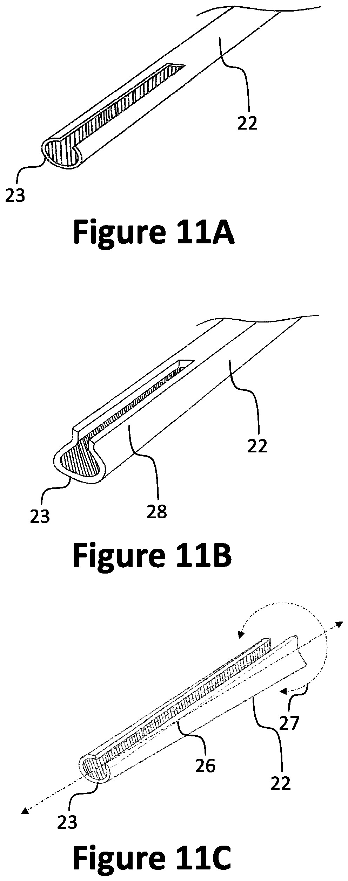

As seen in figure 4, the slit 24 may extend exclusively in the longitudinal direction (e.g. a direction along longitudinal axis 26). However, in other embodiments (see e.g. figure 11C), the slit 24 may also extend in a circumferential direction 27. In some embodiments, the slits may extend in a circumferential and longitudinal direction, e.g. in a helical path around the tubular shape. In further examples, a wall of the shaft 22 may have an oval cross-section as seen in figure 11A. In a further example, as seen in figure 11B, the wall 23 could have a tubular shape and may additionally include a wall 28 or wall segment that extends radially outwardly (or inwardly) from the tubular shape. Thereby, the wall 28 forms a channel which may facilitate sliding the implant 100 into the slit 24. In a further embodiment (see figure 10) the shaft 22 may comprise a second slit 29. The second slit 29 may be at a radially opposing position of the slit 24. The slit 24 and the second slit 29 allow for an easier insertion of the implant 100.

Alternatively to the loading in the shaft described with reference to figure 2A using sutures (see also figure 8C), the implant 100 may be drawn into slit 24 manually or using surgical forceps, in particular when the sutures are attached to an unsuitable edge for pulling the implant into the slit 24. For example, the sutures 102 may be attached to an edge opposing the edge 101 that is slid into slit 24 (as seen in figure 8D) or the sutures 102 may be attached to a distal edge of the implant 100.

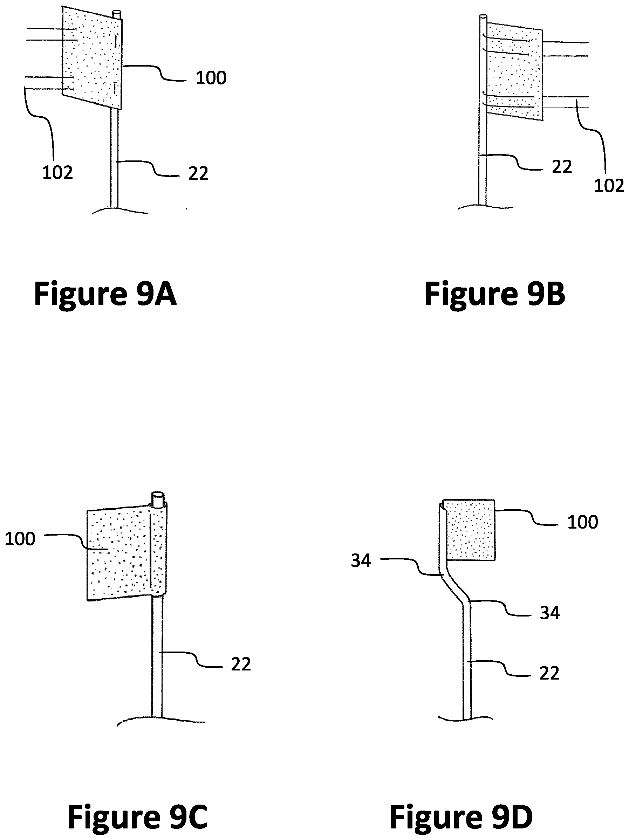

In one alternative embodiment (see figures 9A and 9B) the implant 100 may be fixated to the shaft 22 using the sutures 102. In this case, the sutures 102 may be wound around the shaft 22 or extend through holes in the shaft 22. Thereby, a secure connection to the shaft 22 may be achieved. In a further alternative embodiment (see figure 9C), the implant 100 may extend around the shaft 22 and the edge 101 may be sutured or otherwise attached to another part of the implant 100.

After the implant 100 is loaded to the shaft 22 (within the slit 24 or otherwise), the shaft 22 and the implant 100 are positioned in a proximal portion of the tube 51. The tube 51 includes, as shown in figure 2B, a tube slit 60 that extends from a proximal end of the tube 51. The tube slit 60 may have at least the length of the slit 24 of the shaft 22. Further, the tube slit 60 may have a width that may be wider than a thickness of the implant 100 and may be wider than a diameter of the shaft 22.

Figures 8A and 8B show the insertion tool 50 in greater detail. The tube 51 has a circular cross-section (see figure 8A). Suitable internal diameters ID of the tube 51 may be 1.5 - 19.5 mm, preferably 2.5 - 11.5 mm, and most preferably 5.5 - 9.5 mm. This may allow for rolling of different implant widths. Suitable outer diameters OD of the tube 51 may be 3 - 20mm, preferably 4 -11 mm and most preferably 6.5 - 10.5 mm. These diameters may allow for an arthroscopic or minimally invasive access into the human body.

Although the tube 51 may have a circular shape as shown in figure 8A, it may also be oval or have another shape, e.g. rectangular, quadratic, or polygonal.

The handle 53 extends perpendicular to the longitudinal axis 55 of the tube 51, which may also be described as a pistol grip. The handle 53 may have a longitudinal axis extending perpendicularly to the tube 51. The handle 53 may be arranged in a proximal region of the tube 51. Figure 8B shows a top view of the insertion tool 50. As shown the slit 60 is arranged at an opposing side to the handle 53 of the tube 51, although the slit 60 may also be arranged to any side.

The slit 60 may have a width that is more than a thickness of the implant. In some embodiments the width of the slit 60 is larger than a diameter of the shaft 22. If the width of the slit 60 is larger than a diameter of the shaft 22, a user may insert the shaft 22 with the implant 100 along a radial direction of the tube 51. Otherwise a user may insert the shaft 22 with the implant 100 with a longitudinal movement as the width of the slit 60 may prohibit a radial insertion. In some embodiments, the insertion tool slit widths TSW (see figure 8B) may be 0.5 - 10 mm, preferably 1 - 5 mm and most preferably 2 - 4 mm. A slit length TSL of slit 60 may be 5 - 100 mm, preferably 10 - 50 mm and most preferably 25 - 35 mm. These lengths allow for potential varying implant lengths.

Slit 60 may include a proximal opening 63. The proximal opening 63 may taper, facilitating insertion of the shaft 22 with the implant 100 from the proximal side by guiding the implant 100 into the slit 60 prior to rolling. If the slit 60 includes a distal opening (as e.g. in the second embodiment) the distal opening may taper.

Thereby, the shaft 22 can easily be inserted along the longitudinal axis 55 of the tube 51. A user may hold the delivery tool handle 21 of the delivery device 1 and position the distal end of the shaft 22 in the tube slit 60 as shown in figure 2B.

After positioning the shaft 22 in the tube slit 60, a user may rotate the delivery tool 20 around its own axis as indicated by arrow 62 (figure 2B). The shape of the tube slit 60, more precisely the hollow round inner shape of tube 51 in combination with rotating the delivery tool 20 around its own axis 26 causes the implant 100 to be rolled around the distal tip portion of the shaft 22. Thereby, the sheet-shaped implant 100 can be loaded, giving it a compact form for insertion through an arthroscopic access into the human body. After the implant 100 is rolled around the shaft 22 of the delivery tool 20 it may be delivered.

The position of the implants may be fixated using the sutures 102. As shown, e.g. in figure 1A, the delivery tool handle 21 of the implant delivery tool 20 includes longitudinally extending channels 38. The sutures 102 may be positioned within longitudinally extending channels 38 and wound around bobbin 37. Thereby, the implant 100 is securely held within slit 24. A user may fixate the sutures 102 after fixation of the implant to the shaft 22 (e.g. after the step shown in figure 2A, i.e. after insertion of the implant into the slit 24) or after rolling the implant 100 (e.g. after the step shown in figure 2B).

In a next step (figure 2C) the delivery device, including the implant delivery tool 20 and the insertion tool 50 may be inserted into the human body, e.g. through a cannula 70 as shown in figure 2C. For example, the implant may be fixated to a torn ligament or tendon of a human shoulder joint 200. After passing through the cannula 70, the rolled-up implant 100 may pass through tube 51 and exit the distal end 52 of the tube 51 as shown in figure 2D. For example, a user may hold delivery tool handle 21 and insertion tool handle 53 and push delivery tool handle towards the insertion tool handle 53 thereby passing the implant 100 through tube 51. Of course a user may also pass the rolled-up implant 100 through the tube 51 prior to passing the implant 100 through the cannula 70.

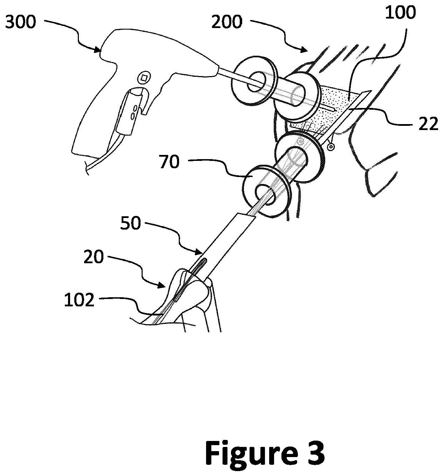

The implant 100 may be unloaded by rotating delivery tool handle 21 around the axis of the shaft 22. For example, as shown in figure 3, a user may position the implant 100 in a suitable position, e.g. on a torn shoulder tendon or ligament, and then rotate delivery tool handle 21. Then, the implant 100 may be fixated on the tendon or ligament. In one particular example, the implant 100 may comprise or is made of a felt material. In this case, the implant may be fixated to the tendon or ligament using a felting device 300 as e.g. shown in any of the following applications: EP 3 968 899, WO 2023/099409, PCT/EP2024/055387, WO 2023/232780, WO 2022/100833. These applications also describe suitable implants for the scope of the present invention.

In some examples, a user may pull sutures 102 in order to position the implant 100 in the body. After the implant 100 has been fixated, the shaft 22 may simply be withdrawn in a distal direction releasing the implant from the slit 24.

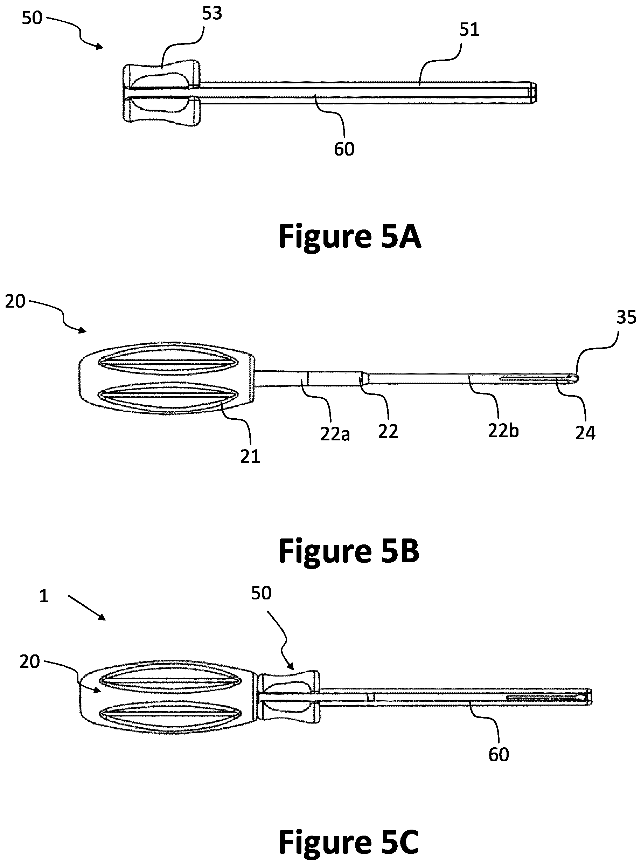

A second embodiment of the present invention can be described with reference to figures 5A to 5C and figures 6A to 7C. In principle, the second embodiment is similar to the first embodiment. Thus, the same reference signs are used and, unless described or shown otherwise, the second embodiment may be the same as the first embodiment. Features of the first and second embodiments may be combined as will be apparent to the skilled person. The second embodiment may have the advantage of improving a usability of the device as compared to the first embodiment. The second embodiment may make loading, unloading and delivery more straightforward and less error-prone. Further, the deployment may be more precisely controlled.

The delivery device 1 according to the second embodiment includes a delivery tool 20 (see figure 5B) and an insertion tool 50 (figure 5A). The insertion tool 50 includes a longitudinally extending tube 51 and a handle 53. The longitudinally extending tube 51 includes a slit 60 that may extend from a proximal end through the entire length of the insertion tool 20 until a distal end of the insertion tool 50. The slit 60 may allow an implant 100 to enter on a distal side of the slit and exit on a proximal side of the slit 60 in a unrolled or partially unrolled configuration. The slit 60 may have a width similar to the width of the first embodiment. Further, the handle 53 extends along a longitudinal direction of the tube 51. As can be seen in figure 5A, the handle 53 may be arranged on a proximal side of the insertion tool 50 and may be round. The slit 60 may also extend through the handle 53.

In the second embodiment, the tube 51 may have a length of 2-25 cm, preferably 7-20 cm, most preferably 11-18 cm. The slit 60 may have a length of 5-30 cm, preferably 10-25 cm, most preferably 14-20 cm. The slit 60 may be longer than the tube since the handle 53 may extend beyond the tube 51 (i.e. along the longitudinal axis 55). The slit width may be the same as in the first embodiments.

The implant delivery tool 20 comprises a handle 21 and a shaft 22 attached to the handle 21. The shaft 22 includes a first portion 22a and a second portion 22b. The first portion 22a may be arranged proximally of the second portion 22b. The first portion 22a may have a larger diameter than the second portion 22b. In some embodiments, an outer diameter of the first portion 22a may correspond to an inner tubular diameter of the tube 51, e.g. in a running fit. Thereby, the delivery tool 20 and the insertion tool 50 may be smoothly rotated (e.g. without wobbling) with respect to each other as described above. This may improve a rolling of the implant 100 (e.g. without the implant slanting or bunching up). Further, the second portion 22b of the shaft 22 has a smaller diameter than then an inner diameter of the tube 51 to allow for an easy insertion of the second portion 22b into the tube 51. The inner diameter of the tube 51 may be larger than the second portion 22b and the implant 100 when wound around the second portion 22b. The second portion 22b may have at least the length of slit 24. In some embodiments, the first portion 22a and the second portion 22b may be connected with a tapering, e.g. a conical tapering as shown in figure 5B.

The slit 24 of the shaft 22 may be similarly formed to the slit 24 described above. In some embodiments, a distal end of the shaft 22 may include an inclined end face 35 (see also figures 1B). The inclined end face 35 may be substantially flat and angled with respect to the longitudinal axis by e.g. 30 to 60°. The inclined end face 35 may facilitate introducing the implant 100 into the slit 24 and/or help advance the shaft 22 through soft tissue during implantation, e.g. by cutting through soft tissue or pushing tissue aside.

Figure 5C shows the delivery tool 20 and the insertion tool 50 in an assembled form, i.e. the insertion tool 50 being slid onto the shaft 22 (similar to figure 1B).

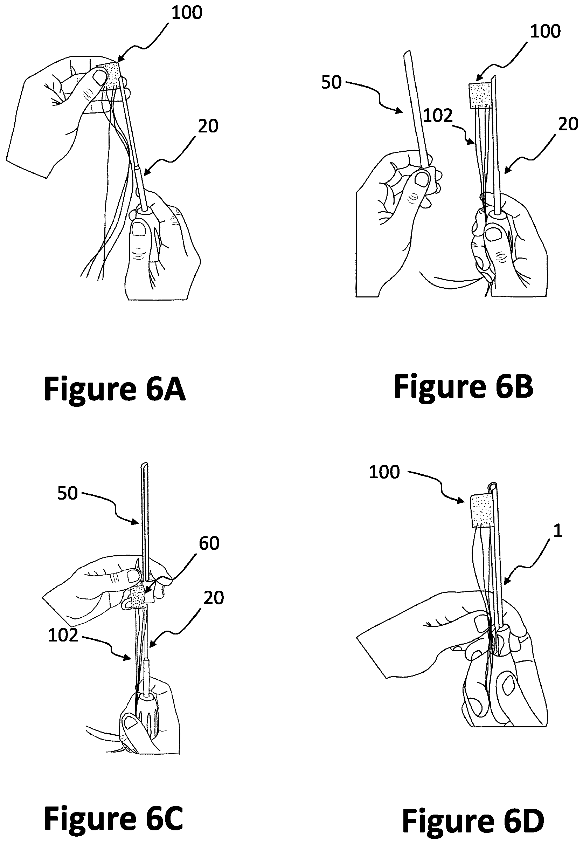

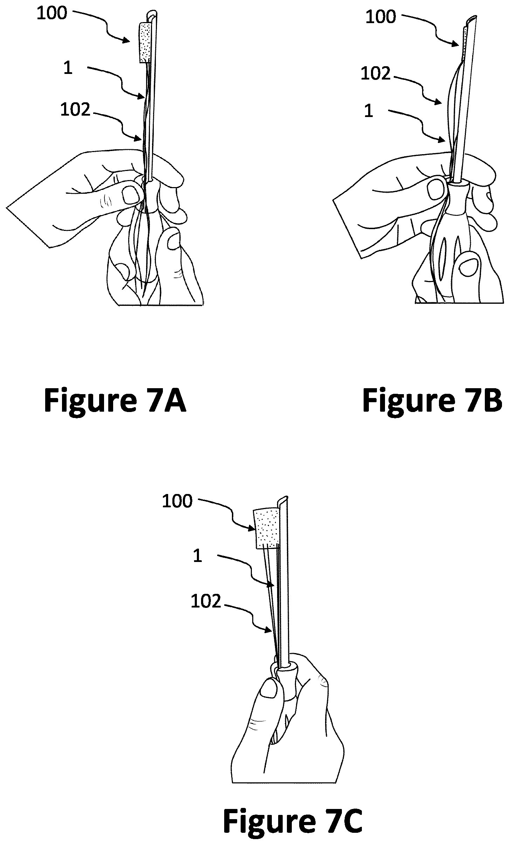

Similarly to the first embodiment, in a first step, the implant 100 is loaded in the slit 24 of the delivery tool 20 (see figure 6A). In the next step, the insertion tool 50 slides onto the shaft 22. In particular, as shown in figures 6B and 6C, a proximal end of the slit 60 is aligned with the implant 100 and then, the implant travels through the slit 60 (see figures 6C and 6D). A user may pull the handle 53 along shaft 22 while also holding handle 21. A user may stop to load the implant 100 in any intermediary position. Preferably, a user pulls the insertion tool 50 until the handle 21 and the handle 53 touch (see figure 7A). Then, the user may rotate the handle 21 relatively to the handle 53, by which the implant 100 is rolled around shaft 22 within the tube 51 (see figures 7A and 7B). Thereby, the implant 100 is given a compact shape and can be transported through a cannula for implantation. The second embodiment allows for a more convenient handling since the axis of tube 51 and shaft 22 are aligned allowing for a quicker and easier rolling. Further, the rolling may be smoother as the tube 51 prevents the shaft 22 from being misaligned with the tube 51.

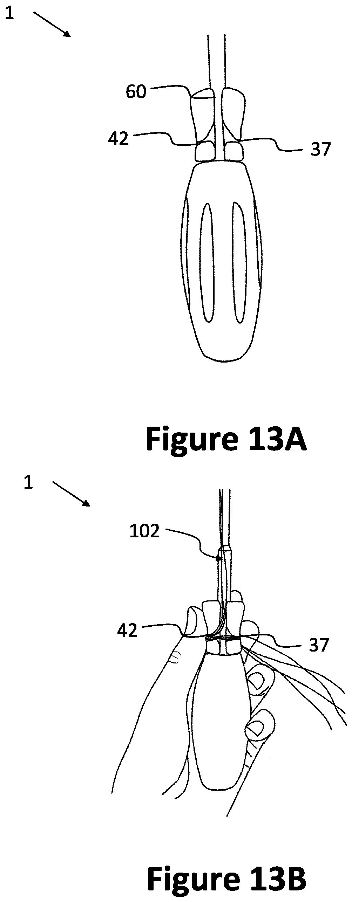

After loading (e.g. rolling the implant 100), a user may fixate the implant in a transport position by holding sutures 102 in the slit (or a further channel) in the handle 53 (see figure 7B). Additionally, handle 53 or handle 21 may include fixation means (e.g. a bobbin or clamp or otherwise for the sutures). As shown in figures 13A and 13B, the handle 53 may include a circumferentially extending channel 42 forming a bobbing 37. The sutures 102 extend through the slit 60 in the handle 53 and may then be wound around the handle 53, securing the implant 100 and the insertion tool 50 in the transport position. A user may directly insert implant 100 into a patient after loading. A user may also prepare the delivery device 1 in advance and only insert the implant 100 into a patient later. Also, a manufacturer may load the implant 100 and package and deliver implant 100 and delivery device 1 including, delivery tool 20 and/or insertion tool 50 as a set that may be ready to use allowing users to directly insert the device 1 into the human body and position the implant 100.

Further, a user may unload a pre-loaded implant 100 in case the user wishes to use a different implant (e.g. different size, different thickness, different material). Then, as described above, the user may load another implant.

Then, similarly to figures 2C and 2D, the implant 100 may be transported through cannula 70. Thereafter, the implant 100 may be released by rotating the handle 53 and the handle 21 with respect to each other forcing the implant out of the tube slit 60 (see figure 7C). As shown in figure 3, the implant 100 may be fixated. To release the implant from the slit 24, the implant delivery tool 20 may be retracted in a proximal direction (e.g. inversely to the loading).

In one embodiment, the shaft 22 may include one or more bends 34 (see figure 9D). This embodiment may be useful in case of complex geometries during implantation. Such shafts 22 may be loaded in the manner described with reference to figures 2A und 2B (without necessarily being implanted as shown with respect to figures 2C to 3).

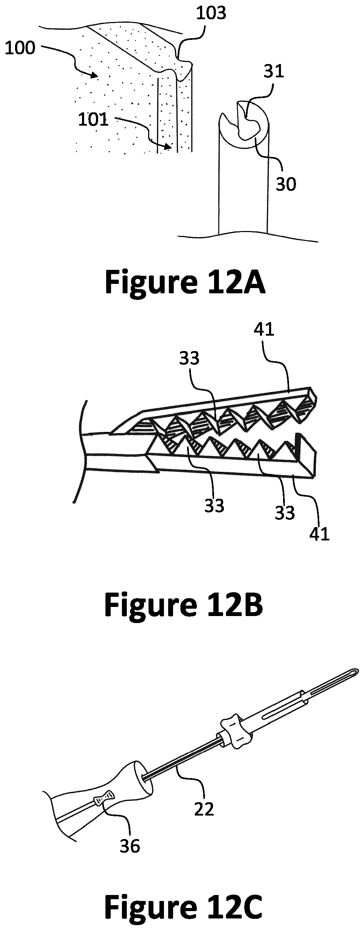

As described above, the slit 24 may open to a circular hollow space (see in particular figure 4). In such embodiments, the edge 101 of the implant 100 may include a bulge, which may prevent inadvertent release of the implant 100, e.g. during rolling. In other embodiments (see figure 12A), the slit 24 may have a U-shape 30 (i.e. in a cross-section, e.g. perpendicular to the longitudinal axis). The slit may have the U-shape over its entire length or only along a portion. The U-shape may improve a grip. Further, the U-shape may include a notch or projection 31 for holding the implant 100. More generally, the slit 60 may form guiding rails for the implant 100.

Correspondingly, the edge 101 of the implant 100 may have a matching shape. For example, as shown in figure 12A, the edge 101 may include a trough 103 that extends along (at least a part of) the edge 101. The edge 101 and the slit 60 may interlock in a form-fit that allows pulling (or pushing) the edge 101 along the longitudinal axis 26 of the shaft 22.

An alternative to the slit 24 is shown in figure 12B. As can be seen, a distal end of the shaft 22 includes forceps 41 that can be actuated to grip the implant 100 at the edge 101. Each of the forceps 41 or only one forceps 41 may include one or more teeth 33 for holding the implant 100. The forceps 41 preferably include opposing teeth 33. Additionally, the handle 21 may include a slider 36 (see figure 12C). The slider 36 may actuate the forceps 41, e.g. using steering wires (not shown).

In certain embodiments (figure 12C), forceps 41 may be provided in addition to the slit 24 described above. For example, shaft 22 may be hollow and forceps 41 may be provided within shaft 22. The forceps 41 may travel along the longitudinal direction 26 inside shaft 22 and may grip an implant 100 positioned distally to the distal end of shaft 22. Then, the implant 100 may be drawn into the slit 24 by pulling forceps 41, which may grip implant 100, in the proximal direction.

Further examples include the following aspects:
1. Delivery device (1) for loading, delivering and/or implanting a flexible sheet-shaped implant (100), the delivery device comprising an implant delivery tool (20) and an insertion tool (50);
   the implant delivery tool (20) including an implant delivery tool handle (21) and a shaft (22), the shaft extending distally from the handle, the shaft being configured to receive an end portion of the sheet-shaped implant;
   the insertion tool (50) including a longitudinally extending tube (51), the longitudinally extending tube having an inner lumen (52), wherein the inner lumen (52) is configured to receive the shaft (22) of the implant delivery tool (20) rotatably, wherein the tube of the insertion tool (50) includes a tube slit (60) that extends from an end of the tube for rolling the sheet-shaped implant (100).
2. Delivery device according to aspect 1, wherein the shaft (22) is defined by a shaft wall (23) and wherein the shaft includes a first longitudinally extending shaft slit (24) in the shaft wall.
3. Delivery device according to aspect 2, wherein the first shaft slit (24) extends from a distal end (25) of the shaft (22).
4. Delivery device according to aspect 2 or 3, wherein the first shaft slit (24) extends exclusively in the longitudinal direction (26) of the shaft (22).
5. Delivery device according to aspect 2 or 3, wherein the first shaft slit (24) extends in a longitudinal (26) and circumferential direction (27) of the shaft, wherein the first shaft slit is in particular curved.
6. Delivery device according to any one of aspects 2 to 5, wherein the shaft wall (23) includes a radially outwardly and/or inwardly extending wall (28) defining a portion of the first shaft slit (24).
7. Delivery device according to any one of aspects 2 to 6, wherein the shaft (22) includes a second shaft slit (29) at a radially opposing side of the first shaft slit.
8. Delivery device according to any one of aspects 2 to 7, wherein the first shaft slit has a U-shape (30).
9. Delivery device according to any one of the aspects 2 to 8, wherein the first shaft slit (24) includes a notch or projection for holding the implant, the notch (31) or projection preferably extending along an axial direction of the shaft.
10. Delivery device according to aspect 1, wherein a distal end portion of the shaft includes a clamping mechanism (32) for gripping the implant.
11. Delivery device according to aspect 10, wherein the clamping mechanism (32) includes one or more teeth (33) for gripping the implant.
12. Delivery device according to any one of the preceding aspects, wherein the tube (51) of the insertion tool (50) extends around the shaft (22) of the implant delivery tool (20) and is arranged to slide on the shaft along a longitudinal axis of the shaft.
13. Delivery device according to any one of the preceding aspects, wherein the shaft and/or the tube (51) comprises or is made of at least one of: stainless steel, nitinol, PEEK, PPSU, or PEI.
14. Delivery device according to any one of the preceding aspects, wherein the insertion tool (50) includes an insertion tool handle (53) for manually gripping the insertion tool.
15. Delivery device according to aspect 14, wherein the insertion tool handle (53) has a body with a round shape for manually gripping and moving the insertion tool.
16. Delivery device according to aspect 14, wherein the insertion tool handle extends in a radial direction (54) or along an axial direction (55) of the tube.
17. Delivery device according to any one of the preceding aspects, wherein an insertion tool handle (53) and/or the implant delivery tool handle (21) comprises or is made of at least one of: silicone, aluminum, stainless steel, carbon fiber, PEEK, PPSU, PEI, PP-HS, POM, PC, and ABS.
18. Delivery device according to any one of the preceding aspects, wherein the shaft (22) includes one or more bends (34).
19. Delivery device according to any one of the preceding aspects, wherein a distal end of the tube and/or a distal end of the shaft includes an inclined end face (35).
20. Delivery device according to any one of the preceding aspects, wherein the insertion tool (50) is connected to the implant delivery tool (20).
21. Delivery device according to one of the preceding aspects, wherein the implant delivery tool handle (21) includes an actuator (36), preferably a slider, and an implant gripper, wherein the actuator is connected to the implant gripper, and wherein actuation of the actuator is configured to cause the gripper to grip the implant and/or move the implant along the longitudinal direction of the shaft.
22. Delivery device according to one of the preceding aspects, wherein a relative position of the tube and the shaft is configured to be locked.
23. Delivery device according to one of the preceding aspects, wherein the implant delivery tool and/or the insertion tool comprises a bobbin (37), preferably at a proximal part, for wrapping sutures connected to the sheet-shaped implant.
24. Delivery device according to one of the preceding aspects, wherein the implant delivery tool handle and/or insertion tool includes a body, the body defining a preferably longitudinal channel for sutures connected to the sheet-shaped implant.
25. Delivery device according to one of the preceding aspects, wherein implant delivery tool or the insertion tool includes a clamp to grip sutures connected to the sheet-shaped implant.
26. Delivery device according to aspect 23 or 25, wherein the bobbin (37) or clamp is connected to delivery device handle or the insertion tool handle with an elastic element, wherein the elastic element is configured to tension sutures connected to the sheet-shaped implant and held by the bobbin or clamp.
27. Delivery device according to one of the preceding aspects, wherein the tube slit (60) of the insertion tool extends from a proximal end (56) of the tube and/or from a distal end (52) of the tube.
28. Delivery device according to one of the preceding aspects, wherein the tube slit (60) extends from the distal end (52) of the tube to the proximal end (56) of the tube.
29. Delivery device according to one of aspects 1 to 27, wherein the tube slit only extends along a portion of the tube length.
30. System including the delivery device according to one of the preceding aspects and a sheet-shaped implant (100).
31. System according to aspect 30, wherein the implant includes a first edge region (101), wherein the edge region includes a notch or projection or trough (103), the notch or projection or trough (103) preferably extending along the edge of the implant.
32. System according to aspect 30 or 31, wherein a shaft slit of the shaft has at least the length of a length and/or width of the implant.
33. Method of loading a flexible sheet-shaped implant into an implant delivery device, comprising the following steps:
   a. Receiving a sheet-shaped implant on a distal end of a shaft of an implant delivery tool; and
   b. Moving the distal end of the shaft with the implant into a tube slit at an end of a tube of an insertion tool; and
   c. Rotating the shaft of the implant delivery tool around its axis relatively to the tube and thereby drawing the implant into the tube through the tube slit and rolling the implant around the shaft.
34. Method according to aspect 33, wherein the receiving step includes receiving the sheet-shaped implant in a shaft slit of the shaft of the implant delivery tool.
35. Method according to aspect 34, wherein the receiving step includes pulling sutures that affixed to the implant to draw the sheet-shaped implant, preferably an edge of the sheet-shaped implant, into the shaft slit.
36. Method according to one of aspects 33 to 35, wherein the receiving step includes pushing or pulling the implant into the shaft slit using a clamp or other tool.
37. Method according to one of aspects 33 to 35, wherein the implant is sutured to the shaft.
38. Method according to one of aspects 33 to 37, wherein the step of moving the distal end of the shaft with the implant into a tube slit comprises sliding the shaft with the implant in the tube until the insertion tool reaches an end position defined by the implant delivery tool, wherein the end position is preferably defined by a handle of the delivery tool.
39. Method according to one of aspects 33 to 38, wherein after rotating the shaft of the implant delivery tool and drawing the implant into the tube, an edge of the implant extends out of the tube.
40. Method according to one of aspects 33 to 39, additionally comprising the step of unloading the sheet-shaped implant, wherein unloading comprises rotating the shaft of the implant delivery tool around its axis relatively to the tube in an opposite direction.
41. Method according to aspect 40, wherein, prior to unloading, the shaft with the implant is pushed through the tube of the insertion tool and wherein a distal portion of the shaft including the implant leaves the tube of the insertion tool.
42. Method according to aspect 40, wherein the step of unloading comprises unloading the implant through the tube slit.

## Claims

1. Delivery device (1) for loading, delivering and/or implanting a flexible sheet-shaped implant (100), the delivery device comprising an implant delivery tool (20) and an insertion tool (50);
the implant delivery tool (20) including an implant delivery tool handle (21) and a shaft (22), the shaft extending distally from the handle, the shaft being configured to receive an end portion of the sheet-shaped implant;
the insertion tool (50) including a longitudinally extending tube (51), the longitudinally extending tube having an inner lumen (52), wherein the inner lumen (52) is configured to receive the shaft (22) of the implant delivery tool (20) rotatably, wherein the tube of the insertion tool (50) includes a tube slit (60) that extends from an end of the tube for rolling the sheet-shaped implant (100).

2. Delivery device according to one of the preceding claims, wherein the tube slit (60) of the insertion tool extends from a proximal end (56) of the tube and/or from a distal end (52) of the tube.

3. Delivery device according to one of the preceding claims, wherein the tube slit (60) extends from the distal end (52) of the tube to the proximal end (56) of the tube.

4. Delivery device according to any one of the preceding claims, wherein the tube (51) of the insertion tool (50) extends around the shaft (22) of the implant delivery tool (20) and is arranged to slide on the shaft along a longitudinal axis of the shaft.

5. Delivery device according to any of the preceding claims, wherein the tube (51) is configured to hold the implant when rolled around the delivery tool.

6. Delivery device according to any one of the preceding claims, wherein the shaft (22) is defined by a shaft wall (23) and wherein the shaft includes a first longitudinally extending shaft slit (24) in the shaft wall.

7. Delivery device according to one of the preceding claims, wherein the implant delivery tool and/or the insertion tool comprises a bobbin (37), preferably at a proximal part, for wrapping sutures connected to the sheet-shaped implant.

8. Delivery device according to one of the preceding claims, wherein the shaft (22) includes a first portion (22a) and second portion (22b), the first portion having a larger diameter than the second portion.

9. Delivery device according to one of the preceding claims, wherein the tube slit (60) includes an opening (62) wherein the opening tapers towards the tube slit.

10. System including the delivery device according to one of the preceding claims and a sheet-shaped implant (100).

11. System according to claim 10, wherein the tube slit has a width that is at least the width of the sheet-shaped implant (100) and/or wherein the tube slit has a length that is at least the length of an edge of the sheet-shaped implant (100).

12. System according to claim 10 or 11, wherein a shaft slit (24) of the shaft (22) has at least the length of a length and/or width of the implant (100).

13. Method of loading a flexible sheet-shaped implant into an implant delivery device, comprising the following steps:
a. Receiving a sheet-shaped implant on a distal end of a shaft of an implant delivery tool; and
b. Moving the distal end of the shaft with the implant into a tube slit at an end of a tube of an insertion tool; and
c. Rotating the shaft of the implant delivery tool around its axis relatively to the tube and thereby drawing the implant into the tube through the tube slit and rolling the implant around the shaft.

14. Method according to claim 13, wherein the receiving step includes receiving the sheet-shaped implant in a shaft slit of the shaft of the implant delivery tool.

15. Method according to one of claims 13 to 14, additionally comprising the step of unloading the sheet-shaped implant, wherein unloading comprises rotating the shaft of the implant delivery tool around its axis relatively to the tube in an opposite direction.
